# EUROPEAN PATENT APPLICATION

(11) **EP 4 529 944 A1**
(43) Date of publication of application: **02.04.2025**
(21) Application number: 24196631.6
(22) Date of filing: 27.08.2024
(51) Int. Cl.: A61M 39/06, A61M 39/10, A61M 5/31, A61M 39/08, A61B 17/34

(54) **ENDOVASCULAR HEMOSTASIS VALVE ADAPTER**

(30) Priority: 28.09.2023 US 202363541024 P; 29.05.2024 US 202418676659
(71) Applicant: Medtronic Vascular Inc., Santa Rosa, CA 95403 (US)
(72) Inventor: DITULLIO, Raymond J., Santa Rosa, 95403 (US)
(74) Representative: Zimmermann & Partner Patentanwälte mbB

(57) **Abstract**

An endovascular hemostasis valve adapter. The adapter includes a proximal face, a distal face, a body extending between the proximal face and the distal face and having a longitudinal axis, and a bypass portion extending away from the distal face and defining a bypass lumen. The proximal face defines a fist sealable opening offset the longitudinal axis and a second sealable opening aligned with the longitudinal axis. The body defines a first lumen extending from the first sealable opening a second lumen extending from the second sealable opening. The first lumen and the second lumen communicate with the bypass lumen. The first sealable opening and the first lumen are configured to sealably pass a first endovascular device. The second sealable opening and the second lumen are configured to sealably pass a second endovascular device.

## Description

### CROSS-REFERENCE TO RELATED APPLICATIONS

This application claims the benefit of U.S. Provisional Patent Application Serial No. 63/541,024, filed September 28, 2023, the entire content of which is incorporated herein by reference.

### TECHNICAL FIELD

The present disclosure relates to endovascular hemostasis valve adapters. One or more of the hemostasis valve adapters may be used in endovascular procedures for delivering and deploying a stent graft branch extension during an iliac branch device procedure.

### BACKGROUND

Endovascular procedures are minimally invasive techniques to deliver clinical treatments in a patient's vasculature. One example of a clinical treatment used in an endovascular procedure is deployment of a stent graft.

A conventional stent graft typically includes a radially expandable reinforcement structure, e.g., formed from a plurality of annular stent rings, and a cylindrically shaped layer of graft material defining a lumen to which the stent rings are coupled. The stent graft is placed inside a patient's vasculature (e.g., blood vessel) to bridge a diseased blood vessel segment (e.g., an aneurismal, dissected, or torn blood vessel segment), and thereby excluding hemodynamic pressures of blood flow from the diseased blood vessel segment.

Challenges may occur in patients with certain types of aneurysms, such as an iliac aneurysm. A cross-over sheath may be used to track an internal iliac bridging device from a contralateral artery, up and over the anatomical bifurcation, through the iliac branch device, and into the iliac artery. One challenge is maintaining hemostasis while parallel devices (e.g., a sheath and one or more guidewires) are used to deploy an iliac stent graft system while preventing or resisting blood loss.

### SUMMARY

In an embodiment, an endovascular hemostasis valve adapter is disclosed. The adapter includes a proximal face, a distal face, a body extending between the proximal face and the distal face and having a longitudinal axis, and a bypass portion extending away from the distal face and defining a bypass lumen. The proximal face defines a fist sealable opening offset the longitudinal axis and a second sealable opening aligned with the longitudinal axis. The body defines a first lumen extending from the first sealable opening a second lumen extending from the second sealable opening. The first lumen and the second lumen communicate with the bypass lumen. The first sealable opening and the first lumen are configured to sealably pass a first endovascular device. The second sealable opening and the second lumen are configured to sealably pass a second endovascular device.

The first sealable opening may include a first one-way valve including a first wide end at the proximal face tapering inward away from the proximal face toward a second narrow end. The second sealable opening includes a second one-way valve including a second wide end at the proximal face tapering inward away from the proximal face toward a second narrow end.

The first lumen may include a first linear portion and an angled portion. The first linear portion may extend from the first sealable opening to the angled portion. The first lumen may further include a second linear portion extending from the angled portion. The second linear portion may be closer to the longitudinal axis of the body than the first linear portion. The second lumen may extend along the longitudinal axis of the body. The first linear portion and/or the second linear portion are parallel to the second lumen. The first lumen and the second lumen may terminate at the bypass lumen. The first sealable opening may have a first diameter and the second sealable opening may have a second diameter. The first diameter may be smaller than the second diameter.

In another embodiment, an endovascular hemostasis valve system is disclosed. The valve system includes a hemostasis valve adapter including a proximal face, a distal face, and a body extending between the proximal face and the distal face. The proximal face defines a first sealable opening and a second sealable opening. The valve system also includes a hub including an outer wall defining an inner cavity including a valve system including a proximal end face. The hemostasis valve adapter may be configured to sealably couple to the hub between the distal face of the hemostasis valve adapter and the proximal end face of the hub such that the first sealable opening is configured to sealably pass a first endovascular device and the second sealable opening is configured to sealably pass a second endovascular device. The valve system may include a single valve system of the distal portion. The endovascular hemostasis valve system may include an introducer sheath system.

The hemostasis valve adapter may include a bypass portion extending away from the distal face and defining a bypass lumen. The valve system may have an unconstrained position and a constrained position. The bypass portion may be configured to sealably couple to the valve system in the constrained position such that the first endovascular device and/or the second endovascular device bypasses the valve system in the constrained position.

The outer wall of the hub may include a proximal region including first screw threads. The hemostasis valve adapter may include a distal outer surface region including second screw threads complimentary to the first screw threads to couple the hemostasis valve adapter and the hub such that the distal face of the hemostasis valve adapter and the proximal face of the hub create a seal therebetween.

In yet another embodiment, an endovascular implant delivery method using an endovascular hemostasis valve is disclosed. The method includes introducing the endovascular hemostasis valve system into a vasculature of a patient over a guidewire using a dilator. The endovascular hemostasis valve system includes a sheath hub including an outer wall including an inner cavity having a valve system including a proximal end face. The endovascular hemostasis valve system may further include a dilator grip connected to the dilator. The method may further include removing the dilator and the dilator grip. The method also includes tracking a hemostasis valve adapter over the guidewire. The hemostasis valve adapter includes a proximal face, a distal face, and a body extending between the proximal face and the distal face and having a longitudinal axis. The proximal face may define a first sealable opening and a second sealable opening. The guidewire tracks over the sealable opening. The method further includes coupling the hemostasis valve adapter to the sheath hub. The method also includes passing an endovascular device through the second sealing opening.

### BRIEF DESCRIPTION OF THE DRAWINGS

Figure 1 depicts partial cross section view of a deployment of a stent graft system within a first common iliac artery (CIA) branching, a first common iliac artery (CIA), and a first internal iliac artery (IIA).
Figure 2 depicts a side view of an introducer sheath system including an introducer sheath and a dilator.
Figure 3 depicts a partial cross section side view of a hub of the introducer sheath system shown in Figure 2.
Figure 4 depicts a side view of the hub of the introducer sheath system shown in Figure 2 where the proximal end of the hub is decoupled from the distal end of the hub.
Figure 5 depicts a side view of the distal end of the hub and a valve adapter where the valve adapter is tracked over a guidewire that extends into the introducer sheath system.
Figure 6 depicts a front view of the valve adapter shown in Figure 5 where the valve adapter defines a central opening and a peripheral opening.
Figure 7 depicts a partial cross section side view of the valve adapter of Figures 5 and 6 coupled to the distal end of the hub and a first guidewire extending therethrough.
Figure 8 depicts a partial cross section side view of the valve adapter of Figures 5 and 6 showing the first guidewire and a second guidewire lumen and a second guidewire extending therethrough.
Figures 9A, 9B, and 9C depict front views of alternative embodiments of valve adapters having alternative port configurations.
Figure 10 depicts a partial cross section side view of a valve adapter sealing to a face of a sheath hub of an introducer sheath system.
Figure 11 depicts a side view of a valve adapter having a Luer port for a first guidewire according to an alternative embodiment.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful.

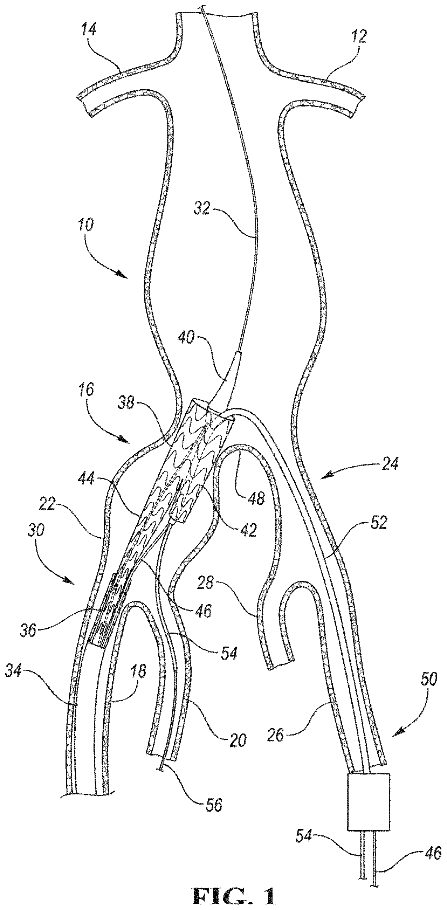

Figure 1 includes a cross section view of abdominal aorta 10. Abdominal aorta 10 branches into left renal artery 12 and right renal artery 14. Abdominal aorta 10 branches into first common iliac artery (CIA) 16, which branches into first external iliac artery (EIA) 18 and first internal iliac artery (IIA) 20. As shown in Figure 1, first CIA 16 includes aneurysm 22. Abdominal aorta 10 also branches into second CIA 24, which branches into second EIA 26 and second IIA 28.

Delivery system 30 is advanced over guidewire 32 through first EIA 18 and into first CIA 16. Delivery system 30 includes sheath 34, stent graft cover 36, asymmetric bifurcated branched stent graft 38, and tapered tip 40. As shown in Figure 1, asymmetric bifurcated branched stent graft 38 is partially deployed with first branch limb 42 fully deployed in first CIA 16 and second branch limb 44 partially deployed within first CIA 16 and first EIA 18.

Throughwire 46 extends through first EIA 18 between sheath 34 and second branch limb 44 and into first branch limb 42. Throughwire 46 extends over aortic bifurcation 48 of abdominal aorta 10 and extends through second CIA 24 and second EIA 26. Guidewire 32 and throughwire 46 may be deployed before deployment of delivery system 30.

Introducer sheath system 50 includes sheath 52, which is advanced over throughwire 46 through second EIA 26 and second CIA 24 and crosses over aortic bifurcation 48 and through first branch limb 42 of asymmetric bifurcated branched stent graft 38. A stent graft branch extension (not shown) configured to extend first branch limb 42 is tracked from a contralateral artery (e.g., second CIA 24) into the artery (e.g., first CIA 16) in which the stent graft branch extension is deployed.

Stent graft cover 54 may be used with introducer sheath system 50. Stent graft cover 54 is configured to cover the stent graft branch extension. Stent graft cover 54 is configured to advance over guidewire 56, which extends through sheath 52 and cannulates into first IIA 20. The stent graft branch extension is deployed by retracting stent graft cover 54 to complete deployment of asymmetrical bifurcated branched stent graft 38.

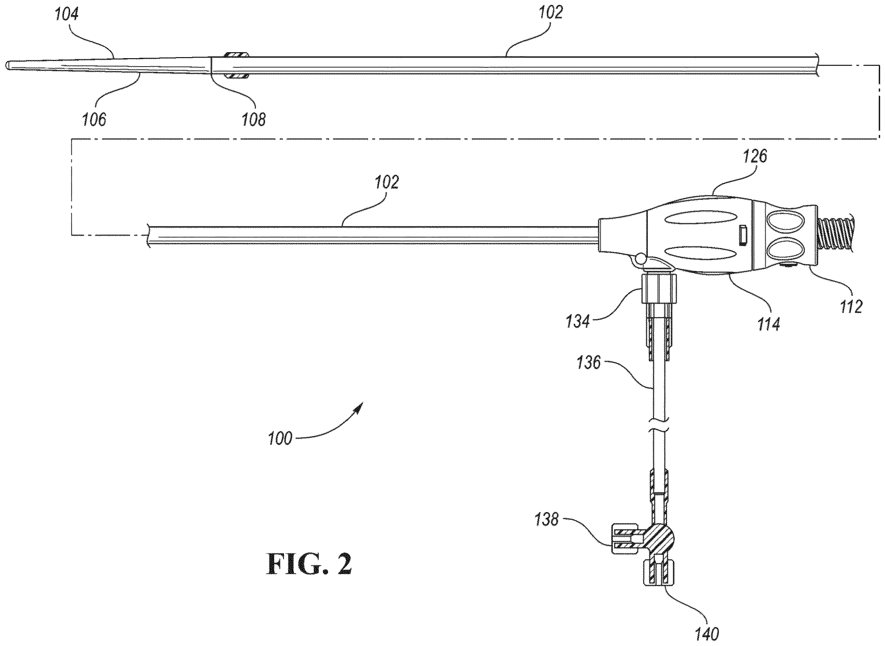

Figure 2 depicts a side view of introducer sheath system 100 including introducer sheath 102 and dilator 104. Introducer sheath system 100 is configured to be utilized with a stent graft delivery system to provide a hemostatic conduit for insertion of one or more endovascular devices while minimizing blood loss associated with endovascular procedures. Introducer sheath 102 may have any of the following sizes or in a range of any two of the following sizes: 4, 4.5, 5, 5.5, 6, 6.5, 7, 7.5, 8, 8.5, 9, 9.5, and 10 French. Introducer sheath 102 may be formed of different lengths (e.g., 28 or 64 cm). Introducer sheath 102 may be reinforced with coiled tubing. Introducer sheath 102 may have a hydrophilic coating. In one or more embodiments, dilator 104 is inserted through the lumen formed by introducer sheath 102 such that distal tip portion 106 of dilator 104 extends beyond the distal end 108 of introducer sheath 102. This insertion operation may occur before introducer sheath system 100 is inserted into the vasculature of the patient. Distal end 108 of introducer sheath 102 may include a radiopaque marker band.

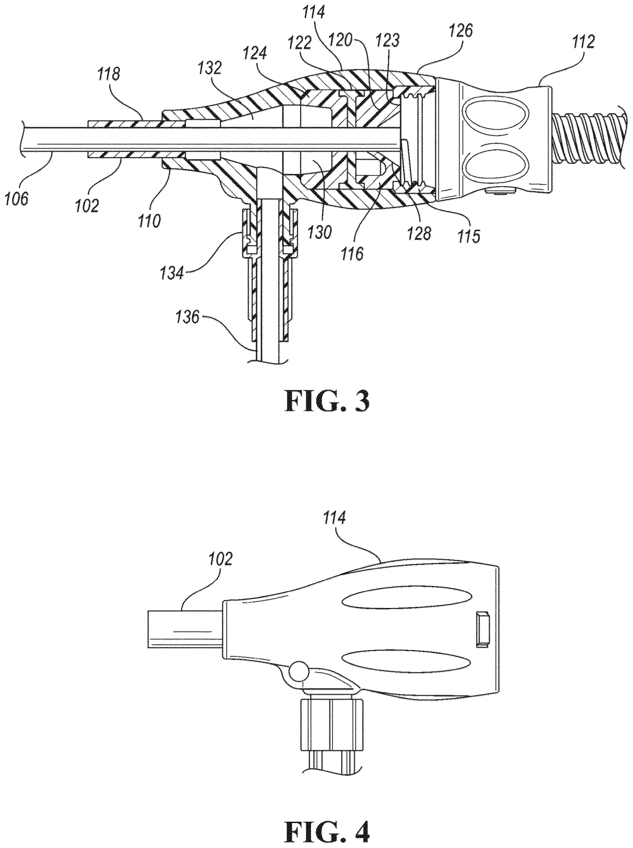

Introducer sheath system includes dilator grip 112 and sheath hub 114 as depicted in Figures 2 and 3. As shown in Figure 3, dilator grip 112 is operatively connected to dilator 106. Dilator grip 112 is configured to provide a grip for manipulating dilator 106. Figure 3 depicts dilator 106 extending from dilator grip 112 through valve system 116 of sheath hub 114 and through introducer sheath 102. As shown in Figure 3, proximal portion 118 of introducer sheath 102 is operatively connected to distal portion 110 of sheath hub 114.

Valve system 116 includes proximal portion 120, middle portion 122, and distal portion 124, which are surrounded by wall 126 of sheath hub 114. Proximal portion 120 extends between distal portion 123 of sheath hub 114 and middle portion 122 of valve system 116. Proximal portion 120 forms a sealing face (further described herein) with distal portion 123 of sheath hub 114 when proximal portion 120 is connected to distal portion 123 (e.g., through threading 115 as shown in Figure 3). Middle portion 122 extends between proximal portion 120 and distal portion 124. Middle portion 122 contacts proximal portion 120 and distal portion 124. Distal portion 124 extends between middle portion 122 and wall 126.

Proximal portion 120, middle portion 122, and/or distal portion 124 of valve system 116 may be formed of a resilient material. A resilient material may be used to form a component of a first shape that can be elastically deformed into a second, different shape. For example, proximal portion 120, middle portion 122, and/or distal portion 124 of valve system may have the first shape when unconstrained (e.g., dilator 106 is not extending through valve system 116) and may have the second shape when constrained (e.g., dilator 106 extends through valve system 116). The proximal portion 120, middle portion 122, and/or distal portion 124 returns to the first shape or substantially its first shape when unconstrained again (e.g., when dilator 106 is withdrawn from valve system 116). The resilient material may be an elastomeric material. Proximal portion 120, middle portion 122, and/or distal portion 124 of valve system 116 may be formed of a stretchable material.

Valve system 116 may be a one-way valve system configured to prevent or resist the flow of blood in one direction. The one-way valve system may be configured to prevent or resist the back flow of blood through valve system 116 and out of sheath hub 114 of introducer sheath system 100, thereby preventing or resisting the loss of blood through introducer sheath system 100 during an endovascular medical procedure (e.g., introduction of a stent graft branch extension from a contralateral artery). Valve system 116 may be referred to as a hemostasis valve system.

As shown in Figure 3, proximal portion 120 of valve system 116 defines cavity 128. In an unconstrained state (not shown in Figure 3), cavity 128 includes a wide end extending from dilator grip 112, for example. The wide end tapers inward away from dilator grip 112 into an apex, thereby forming a conical shape. In a constrained state (as shown in Figure 3), the apex of cavity 128 widens to accommodate dilator 106 (or other medical components as described herein). Valve system 116 defining cavity 128 may be referred to as a duck bill type valve, which permits insertion of medical components from a proximal to distal direction while preventing or resisting blood flow in a distal to proximal direction. Valve system 116 also includes middle portion 122, which may be an annular seal to enhance the seal within valve system 116 and a medical component (e.g., dilator 106) inserted into valve system 116. Valve system 116 also includes distal portion 124, which may be a seal forming cavity 130 to communicate with cavity 132 formed in a portion of wall 126.

Introducer sheath system 100 includes sidearm 134 extending from sheath hub 114. Sidearm 134 may define a sidearm lumen at least partially extending through sidearm 134. Sidearm tube 136 may extend within the sidearm lumen to operatively connect sidearm tube 136 to sidearm 134. A clinician may introduce a fluid through sidearm tube 136 and the sidearm lumen, which communicates with cavity 132, to flush at least a portion of sheath hub 114. One or more fluids may be introduced through first flush port 138 and/or second flush port 140 using a three-way valve, for example.

Dilator 106 may track a guidewire (e.g., throughwire 46 shown in Figure 1) as dilator 106 extends through introducer sheath 102. The guidewire may have any of the following diameters or in a range of any two of the following diameters: 0.030, 0.031, 0.032, 0.033, 0.034, 0.035, 0.036, 0.037, 0.038, 0.039, and 0.040 inches. Dilator 106 may be configured to provide a smooth transition of introducer sheath 102 into the vasculature of a patient. Dilator 106 and introducer sheath 102 may be advanced together over the guidewire as the guidewire is grasped to provide relative movement of dilator 106 and introducer 102 over the guidewire. After introducer sheath 102 is fully inserted (e.g., as shown relative to introducer sheath 52 shown in Figure 1), dilator 106 is removed from introducer sheath 102.

After dilator 106 is removed from introducer sheath 102, dilator grip 112 may be decoupled (e.g., unscrewed) from sheath hub 114. Figure 4 depicts dilator grip 112 decoupled from sheath hub 114.

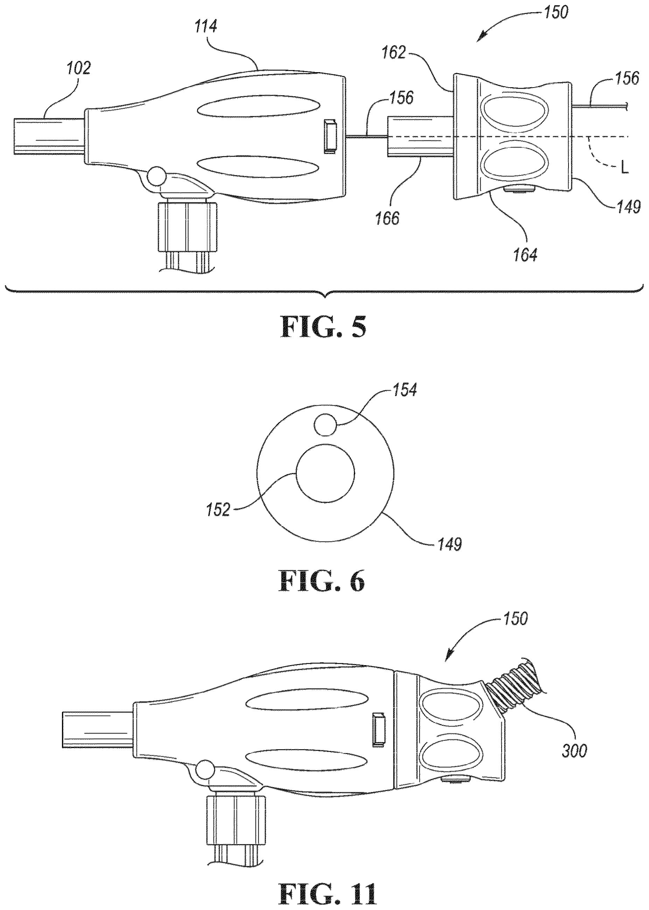

Figure 5 depicts a side view of sheath hub 114 and valve adapter 150. Figure 6 depicts a front view of proximal face 149 of valve adapter 150 defining central opening 152 and peripheral opening 154. Valve adapter 150 is tracked over first guidewire 156 that extends into introducer sheath system 100 or other endovascular implant delivery system.

As shown in Figure 11, Luer port 300 is connected to valve adapter 150. Luer port 300 may be aligned with peripheral opening 154 such that valve adapter 150 may be tracked over first guidewire 156 through Luer port 300. Luer port 300 may be include a first connector and valve adapter 150 may include a second connector where the first and second connectors may be coupled to create a connection that prevents or resists leaking. One of the first or second connectors may be a male connector and the other of the first or second connector may be a female connector.

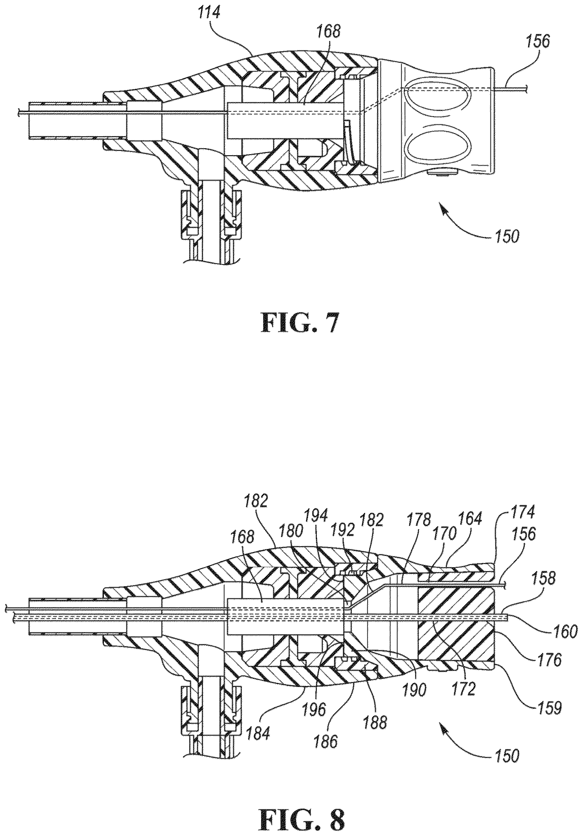

Figure 7 depicts a partial cross section side view of valve adapter 150 coupled to sheath hub 114 and first guidewire 156 extending therethrough. Figure 8 depicts a partial cross section side view of valve adapter 150 showing first guidewire 156 and second guidewire lumen 158 and second guidewire 160 extending therethrough.

With reference to Figures 5 through 8, valve adapter 150 includes proximal face 149 and distal face 162 and body 164 extending between proximal face 149 and distal face 162. Body 164 extends along longitudinal axis L. Valve adapter 150 also includes bypass portion 166 extending away from distal face 162 and defining bypass lumen 168.

In one or more embodiments, proximal face 149 defines central opening 152 and peripheral opening 154. In one or more embodiments, central opening 152 and peripheral opening 154 are sealable openings such that blood loss is prevented or resisted when introducer sheath system 100 is deployed. Central opening 152 and peripheral opening 154 may be configured to permit passage of one or more endovascular devices therethrough while maintaining hemostasis across the valve. Central opening 152 may be aligned with longitudinal axis L and peripheral opening 154 may be offset longitudinal axis L. In one or more embodiments, peripheral opening 154 has a first diameter and central opening has a second diameter larger than the first diameter.

As shown in Figure 8, body 164 defines first lumen 170 extending from peripheral opening 154. As further shown in Figure 8, body defines second lumen 172 extending from central opening 152. In one or more embodiments, first lumen 170 and second lumen 172 communicate with bypass lumen 168. Peripheral opening 154 and first lumen 170 are configured to sealably pass a first endovascular device (e.g., first guidewire 156). Central opening 152 and second lumen 172 are configured to sealably pass a second endovascular device (e.g., catheter, stent-graft delivery system, second guidewire lumen 158 and/or second guidewire 160).

Peripheral opening 154 may include first one-way valve 174 including a first wide end at proximal face 159 tapering inward away from proximal face 159 toward a second narrow end. First one-way valve 174 may be configured to permit passage of one or more endovascular devices therethrough while maintaining hemostasis across the first one-way valve 174. Central opening 152 may include second one-way valve 176 including a first wide end at proximal face 159 tapering inward away from proximal face 159 toward a second narrow end. Second one-way valve 176 may be configured to permit passage of one or more endovascular devices therethrough while maintaining hemostasis across the second one-way valve 176.

As shown in Figure 8, first lumen 170 includes first linear portion 178, second linear portion 180, and angled portion 182 extending between first linear portion 178 and second linear portion 180. First linear portion 178 may extend from peripheral opening 154 to angled portion 182. Second linear portion 180 may extend from angled portion 182 to distal face 162 of valve adapter 150. This configuration of first lumen 170 may be used to provide a separate lumen for one or more first endovascular devices while maintaining communication between peripheral opening 154 and bypass lumen 168. In one or more embodiments, second linear portion 180 is closer to longitudinal axis L of body 164 than first linear portion 178. Second lumen 172 may extend along longitudinal axis L of body 164. First linear portion 178 and/or second linear portion 180 may be parallel to second lumen 172. In one or more embodiments, first lumen 170 and second lumen 172 terminate at bypass lumen 168.

Outer wall 182 of sheath hub 184 may include proximal region 186 including first screw threads 188. Valve adapter 150 includes distal outer surface region 190 including second screw threads 192 complimentary to first screw threads 188. In at least one embodiment, valve adapter may use the identical attachment mechanism as the dilator grip 112, or a functionally interchangeable mechanism. For example, the second screw threads 192 of the valve adapter 150 may the identical or functionally interchangeable with the screw threads of dilator grip 112. Accordingly, after the dilator grip has been removed, the valve adapter 150 may replace the dilator grip using the same mechanism of attachment that the dilator grip used to connect to the hub. First screw threads 188 and second screw threads 192 are configured to couple valve adapter 150 and sheath hub 184 such that distal face 194 of valve adapter 150 and proximal face 196 of sheath hub 184 seal to each other. Alternatively, first screw threads 188 may be a first connector and second screw threads 192 may be a second connector. The first and second connectors may be coupled to create a connection that prevents or resists leaking. One of the first or second connectors may be a male connector and the other of the first or second connector may be a female connector.

As shown in Figure 6, peripheral opening 154 is situated at a twelve o'clock position around the periphery of proximal face 149. In other embodiments, the peripheral opening may be positioned at different locations around the periphery of proximal face 149 (e.g., three o'clock, six o'clock, or nine o'clock).

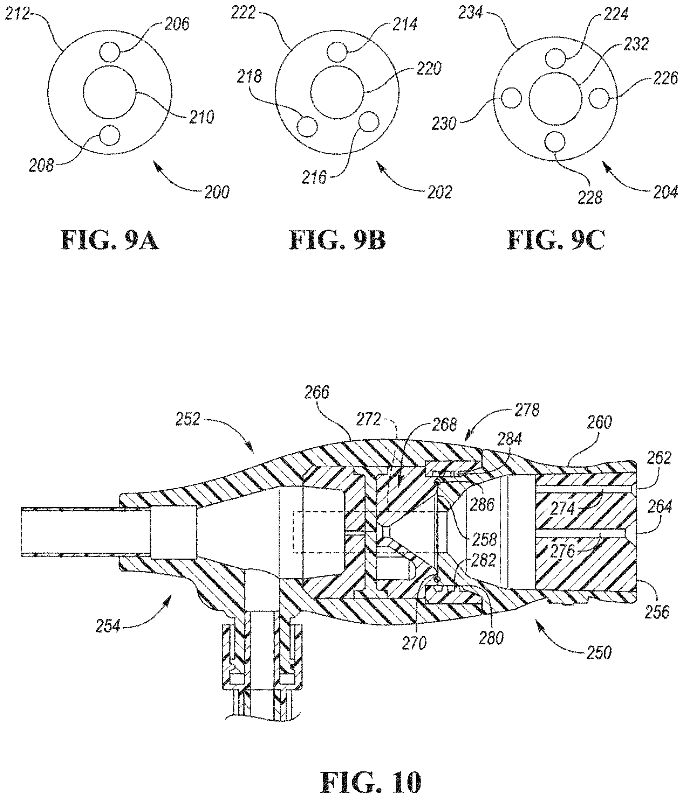

Figures 9A, 9B, and 9C depict front views of alternative embodiments of valve adapters 200, 202, and 204 respectively having alternative peripheral opening configurations. Valve adapter 200 includes first and second peripheral openings 206 and 208 and central opening 210. As shown in Figure 9A, first peripheral opening 206 is located at a twelve o'clock position around the periphery of proximal face 212 and second peripheral opening 208 is located at a six o'clock position around the periphery of proximal face 212 (e.g., opposite each other). In other embodiments, the peripheral openings may be positioned at different locations around the periphery of proximal face 212 (e.g., three o'clock and nine o'clock, respectively). The configuration shown in Figure 9A may be utilized to accommodate the passage of three or more endovascular devices. First and second peripheral openings 206 and 208 may communicate with first and second adapter lumens, respectively, which are in communication with an adapter bypass lumen such that endovascular devices may pass through the adapter and into the vasculature of a patient. First and second adapter lumens may each be substantially similar to first lumen 170, described above.

Valve adapter 202 shown in Figure 9B includes first, second, and third peripheral openings 214, 216, and 218 and central opening 220. As shown in Figure 9B, first peripheral opening 214 is located at a twelve o'clock position around the periphery of proximal face 222, second peripheral opening 216 is located at about a five o'clock position around the periphery of proximal face 222, and third peripheral opening 218 is located at about a seven o'clock position around the periphery of proximal face 222. In other embodiments, the peripheral openings may be positioned at different locations around the periphery of proximal face 222 (e.g., one o'clock, six o'clock, and eleven o'clock). The peripheral openings may be spaced equally about the central opening, such as about 120 degrees from each other. The configuration shown in Figure 9B may be utilized to accommodate the passage of four or more endovascular devices. First, second, and third peripheral openings 214, 216, and 218 may communicate with first, second, and third adapter lumens, respectively, which are in communication with an adapter bypass lumen such that endovascular devices may pass through the adapter and into the vasculature of a patient. First, second, and third adapter lumens may each be substantially similar to first lumen 170, described above.

Valve adapter 204 shown in Figure 9C includes first, second, third, and fourth peripheral openings 224, 226, 228, and 230 and central opening 232. As shown in Figure 9C, first peripheral opening 224 is located at a twelve o'clock position around the periphery of proximal face 234, second peripheral opening 226 is located at a three o'clock position around the periphery of proximal face 234, third peripheral opening 228 is located at a six o'clock position around the periphery of proximal face 234, and fourth peripheral opening 230 is located at a nine o'clock position around the periphery of proximal face 234 (e.g., equally spaced about the center opening). In other embodiments, the peripheral openings may be positioned at different locations around the periphery of proximal face 234 (e.g., one-thirty, four-thirty, seven-thirty, and ten-thirty). The configuration shown in Figure 9C may be utilized to accommodate the passage of five or more endovascular devices. First, second, third, and fourth peripheral openings 224, 226, 228, and 230 may communicate with first, second, third, and fourth adapter lumens, respectively, which are in communication with an adapter bypass lumen such that endovascular devices may pass through the adapter and into the vasculature of a patient. First, second, third, and fourth adapter lumens may each be substantially similar to first lumen 170, described above.

Figure 10 depicts a partial cross section side view of valve adapter 250 sealing to sheath hub 252 of introducer sheath system 254. Valve adapter 250 includes proximal face 256, distal face 258, and body 260 extending between proximal face 256 and distal face 258. Valve adapter 250 may form a single valve (e.g., valve adapter 250 only includes a single valve configured to accept multiple endovascular devices through a bypass lumen as described herein). Proximal face 256 defines first sealable opening 262 and second sealable opening 264. Sheath hub 252 includes outer wall 266 defining an inner cavity including valve system 268 having proximal end face 270. Valve adapter 250 is configured to sealably couple to sheath hub 252 between distal face 258 of valve adapter 250 and proximal end face 270 of sheath hub 252 such that first sealable opening 262 is configured to sealably pass a first endovascular device (e.g., guidewire, guidewire lumen, sheath, cover, implant, etc.) and second sealable opening 264 is configured to sealably pass a second endovascular device.

As shown in Figure 10, valve system 268 is in an unconstrained state (e.g., valve system 268 is not deformed or stretched). As shown in dotted lines, valve adapter 250 may include bypass portion 272 extending away from distal face 258 and defining a bypass lumen. When bypass portion 272 is inserted into valve system 268, valve system 268 transitions from the unconstrained state to a constrained state to accommodate bypass portion 272 thereby sealing to valve system 268. Bypass lumen communicates with first and second adapter lumens 274 and 276 extending from first and second sealable openings 262 and 264 such that first and/or second endovascular devices may extend therethrough and bypass valve system 268.

Outer wall 266 of sheath hub 252 may include proximal region 278 including first screw threads 280. Valve adapter 250 includes distal outer surface region 282 including second screw threads 284 complimentary to first screw threads 280. In at least one embodiment, valve adapter may use the identical attachment mechanism as the dilator grip 112, or a functionally interchangeable mechanism. For example, the second screw threads 284 of the valve adapter 250 may the identical or functionally interchangeable with the screw threads of dilator grip 112. Accordingly, after the dilator grip has been removed, the valve adapter 250 may replace the dilator grip using the same mechanism of attachment that the dilator grip used to connect to the hub. First screw threads 280 and second screw threads 284 are configured to couple valve adapter 250 and sheath hub 254 such that distal face 258 of valve adapter 250 and proximal face 270 of sheath hub 252 seal to each other. Alternatively, first screw threads 280 may be a first connector and second screw threads 284 may be a second connector. The first and second connectors may be coupled to create a connection that prevents or resists leaking. One of the first or second connectors may be a male connector and the other of the first or second connector may be a female connector.

As shown in Figure 10, sealing member 286 (e.g., an O-ring or gasket) is located between distal face 258 and proximal face 270 to improve the seal therebetween. Sealing member 286 may be circular shaped and formed of an elastomeric or stretchable material. Sealing member 286 may be attached to distal face 258 or proximal face 270 before they are brought together to form a seal.

In one or more embodiments, an endovascular implant delivery method using an endovascular hemostasis valve system is disclosed. For example, the endovascular hemostasis valve system may be introducer sheath system 100. Introducer sheath system 100 may be introduced into a vasculature of a patient over a guidewire using dilator 106. Once introducer sheath is placed within the patient's vasculature, dilator 106 and dilator grip 112 may be removed from introducer sheath system 100 while maintaining a guidewire (e.g., guidewire 156) within sheath hub 114. Valve adapter 150 (or 250) may be tracked over the guidewire (e.g., through peripheral opening 154) and then valve adapter 150 may be coupled to sheath hub 114. The coupling step may include sealing a bypass portion (e.g., bypass portion 166) to valve system (e.g., valve system 268) in a constrained position. An endovascular device may then be passed through central opening 152 while maintaining hemostasis. The passing step may include passing the endovascular device through the by the bypass portion. By replacing the dilator grip 112 with a valve adapter (150, 250), the sheath system may go from properly sealing against one medical device (e.g., guidewire, catheter, delivery system, etc.) to properly sealing against two or more medical devices (e.g., any combination of guidewires, catheters, delivery systems, etc.). By incorporating the same, or functionally equivalent, attachment mechanism, the valve adapter may leverage the connection mechanism already present in the hub to ensure an easy and secure seal therewith. The valve adapter may therefore provide improved sealing for procedures requiring multiple devices to be inserted through a single sheath.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

Further disclosed herein is the subject matter of the following clauses:
1. An endovascular hemostasis valve adapter comprising:
   a proximal face;
   a distal face;
   a body extending between the proximal face and the distal face and having a longitudinal axis; and
   a bypass portion extending away from the distal face and defining a bypass lumen,
   the proximal face defining a first sealable opening offset the longitudinal axis and a second sealable opening aligned with the longitudinal axis, the body defining a first lumen extending from the first sealable opening and a second lumen extending from the second sealable opening, the first lumen and the second lumen communicating with the bypass lumen, the first sealable opening and the first lumen configured to sealably pass a first endovascular device, and the second sealable opening and the second lumen configured to sealably pass a second endovascular device.
2. The endovascular hemostasis valve adapter of clause 1, wherein the first sealable opening includes a first one-way valve including a first wide end at the proximal face tapering inward away from the proximal face toward a second narrow end.
3. The endovascular hemostasis valve adapter of clause 1 or 3, wherein the second sealable opening includes a second one-way valve including a second wide end at the proximal face tapering inward away from the proximal face toward a second narrow end.
4. The endovascular hemostasis valve adapter of clause 1 or of any preceding clause, wherein the first lumen includes a first linear portion and an angled portion, the first linear portion extending from the first sealable opening to the angled portion.
5. The endovascular hemostasis valve adapter of clause 4 or of any preceding clause, wherein the first lumen further includes a second linear portion extending from the angled portion.
6. The endovascular hemostasis valve adapter of clause 5 or of any preceding clause, wherein the second linear portion is closer to the longitudinal axis of the body than the first linear portion.
7. The endovascular hemostasis valve adapter of clause 5 or of any preceding clause, wherein the second lumen extends along the longitudinal axis of the body.
8. The endovascular hemostasis valve adapter of clause 7 or of any preceding clause, wherein the first linear portion and/or the second linear portion are parallel the second lumen.
9. The endovascular hemostasis valve adapter of clause 1 or of any preceding clause, wherein the first lumen and the second lumen terminate at the bypass lumen.
10. The endovascular hemostasis valve adapter of clause 1 or of any preceding clause, wherein the first sealable opening has a first diameter and the second sealable opening has a second diameter, and the first diameter is smaller than the second diameter.
11. An endovascular hemostasis valve system comprising:
   a hemostasis valve adapter including a proximal face, a distal face, and a body extending between the proximal face and the distal face, the proximal face defining a first sealable opening and a second sealable opening; and
   a hub including an outer wall defining an inner cavity including a valve system including a proximal end face,
   the hemostasis valve adapter configured to sealably couple to the hub between the distal face of the hemostasis valve adapter and the proximal end face of the hub such that the first sealable opening is configured to sealably pass a first endovascular device and the second sealable opening is configured to sealably pass a second endovascular device.
12. The endovascular hemostasis valve system of clause 11 or of any preceding clause, wherein the hemostasis valve adapter includes a bypass portion extending away from the distal face and defining a bypass lumen, the valve system has an unconstrained position and a constrained position, the bypass portion is configured to sealably couple to the valve system in the constrained position such that the first endovascular device and/or the second endovascular device bypasses the valve system in the constrained position.
13. The endovascular hemostasis valve system of clause 11 or of any preceding clause, wherein the outer wall of the hub includes a proximal region including first screw threads, the hemostasis valve adapter includes a distal outer surface region including second screw threads complimentary to the first screw threads to couple the hemostasis valve adapter and the hub such that the distal face of the hemostasis valve adapter and the proximal face of the hub create a seal therebetween.
14. The endovascular hemostasis valve system of clause 11 or of any preceding clause, wherein the valve system is a single valve system of the distal portion.
15. The endovascular hemostasis valve system of clause 11 or of any preceding clause, wherein the endovascular hemostasis valve system includes an introducer sheath system.
16. An endovascular implant delivery method using an endovascular hemostasis valve system, the method comprising:
   introducing the endovascular hemostasis valve system into a vasculature of a patient over a guidewire using a dilator, the endovascular hemostasis valve system includes a sheath hub including an outer wall defining an inner cavity having a valve system including a proximal end face, the endovascular hemostasis valve system further includes a dilator grip connected to the dilator;
   removing the dilator and the dilator grip;
   tracking a hemostasis valve adapter over the guidewire, the hemostasis valve adapter including a proximal face, a distal face, and a body extending between the proximal face and the distal face and having a longitudinal axis, the proximal face defining a first sealable opening and a second sealable opening, the guidewire tracking over the first sealable opening;
   coupling the hemostasis valve adapter to the sheath hub; and
   passing an endovascular device through the second sealable opening.
17. The endovascular implant delivery method of clause 16 or of any preceding clause, wherein the hemostasis valve adapter includes a bypass portion extending away from the distal face and defining a bypass lumen, the valve system having an unconstrained position and a constrained position, the coupling step includes sealing the bypass portion to the valve system in the constrained position, and the passing step includes passing the endovascular device through the bypass lumen.
18. The endovascular implant delivery method of clause 16 or of any preceding clause, wherein the outer wall of the introducer hub includes a proximal region including first screw threads, the hemostasis valve adapter includes a distal outer surface region including second screw threads complimentary to the first screw threads, the dilator grip includes a distal outer surface region including third screw threads complimentary to the first screw threads, the coupling step includes coupling the first screw threads to the second screw threads.
19. The endovascular implant delivery method of clause 16 or of any preceding clause, wherein the first sealable opening is offset the longitudinal axis and the second sealable opening is aligned with the longitudinal axis.
20. The endovascular implant delivery method of clause 16 or of any preceding clause, wherein the coupling step includes sealing the distal face of the hemostasis valve adapter to the proximal end face of the hemostasis valve system.

Further disclosed herein is an endovascular hemostasis valve adapter. The adapter includes a proximal face, a distal face, a body extending between the proximal face and the distal face and having a longitudinal axis, and a bypass portion extending away from the distal face and defining a bypass lumen. The proximal face defines a fist sealable opening offset the longitudinal axis and a second sealable opening aligned with the longitudinal axis. The body defines a first lumen extending from the first sealable opening a second lumen extending from the second sealable opening. The first lumen and the second lumen communicate with the bypass lumen. The first sealable opening and the first lumen are configured to sealably pass a first endovascular device. The second sealable opening and the second lumen are configured to sealably pass a second endovascular device.

## Claims

1. An endovascular hemostasis valve adapter comprising:
a proximal face;
a distal face;
a body extending between the proximal face and the distal face and having a longitudinal axis; and
a bypass portion extending away from the distal face and defining a bypass lumen,
the proximal face defining a first sealable opening offset the longitudinal axis and a second sealable opening aligned with the longitudinal axis, the body defining a first lumen extending from the first sealable opening and a second lumen extending from the second sealable opening, the first lumen and the second lumen communicating with the bypass lumen, the first sealable opening and the first lumen configured to sealably pass a first endovascular device, and the second sealable opening and the second lumen configured to sealably pass a second endovascular device.

2. The endovascular hemostasis valve adapter of claim 1, wherein the first sealable opening includes a first one-way valve including a first wide end at the proximal face tapering inward away from the proximal face toward a second narrow end.

3. The endovascular hemostasis valve adapter of claim 1 or 2, wherein the second sealable opening includes a second one-way valve including a second wide end at the proximal face tapering inward away from the proximal face toward a second narrow end.

4. The endovascular hemostasis valve adapter of any preceding claim, wherein the first lumen includes a first linear portion and an angled portion, the first linear portion extending from the first sealable opening to the angled portion.

5. The endovascular hemostasis valve adapter of claim 4, wherein the first lumen further includes a second linear portion extending from the angled portion.

6. The endovascular hemostasis valve adapter of claim 5, wherein the second linear portion is closer to the longitudinal axis of the body than the first linear portion.

7. The endovascular hemostasis valve adapter of any preceding claims, wherein the second lumen extends along the longitudinal axis of the body.

8. The endovascular hemostasis valve adapter of any one of claims 4 to 7, wherein the first linear portion and/or the second linear portion are parallel the second lumen.

9. The endovascular hemostasis valve adapter of any preceding claim, wherein the first lumen and the second lumen terminate at the bypass lumen.

10. The endovascular hemostasis valve adapter of any preceding claim, wherein the first sealable opening has a first diameter and the second sealable opening has a second diameter, and the first diameter is smaller than the second diameter.

11. An endovascular hemostasis valve system comprising:
a hemostasis valve adapter including a proximal face, a distal face, and a body extending between the proximal face and the distal face, the proximal face defining a first sealable opening and a second sealable opening; and
a hub including an outer wall defining an inner cavity including a valve system including a proximal end face,
the hemostasis valve adapter configured to sealably couple to the hub between the distal face of the hemostasis valve adapter and the proximal end face of the hub such that the first sealable opening is configured to sealably pass a first endovascular device and the second sealable opening is configured to sealably pass a second endovascular device.

12. The endovascular hemostasis valve system of claim 11, wherein the hemostasis valve adapter includes a bypass portion extending away from the distal face and defining a bypass lumen, the valve system has an unconstrained position and a constrained position, the bypass portion is configured to sealably couple to the valve system in the constrained position such that the first endovascular device and/or the second endovascular device bypasses the valve system in the constrained position.

13. The endovascular hemostasis valve system of claim 11 or 12, wherein the outer wall of the hub includes a proximal region including first screw threads, the hemostasis valve adapter includes a distal outer surface region including second screw threads complimentary to the first screw threads to couple the hemostasis valve adapter and the hub such that the distal face of the hemostasis valve adapter and the proximal face of the hub create a seal therebetween.

14. The endovascular hemostasis valve system of any one of claims 11 to 13, wherein the valve system is a single valve system of the distal portion.

15. The endovascular hemostasis valve system of any one of claims 11 to 14, wherein the endovascular hemostasis valve system includes an introducer sheath system.
